# EUROPEAN PATENT SPECIFICATION

(11) **EP 2 695 600 B1**
(45) Date of publication and mention of the grant of the patent: **01.02.2017**
(21) Application number: 12425137.2
(22) Date of filing: 09.08.2012
(51) Int. Cl.: A61K 8/49, A61K 8/60, A61Q 19/00

(54) **Dermal cosmetic compositions having osmoprotecting activity**
Hautkosmetikzusammensetzungen mit Osmoprotektionsaktivität
Compositions cosmétiques dermiques ayant une activité osmo-protectrice

(43) Date of publication of application: 12.02.2014
(73) Proprietor: Unifarco S.p.A., 32035 Santa Giustina (BL) (IT)
(72) Inventor: Baratto, Giovanni, 32035 Santa Giustina BL (IT)
(74) Representative: Asensio, Raffaella Consuelo

(56) References cited:
- EP-A1- 0 671 161
- EP-A2- 0 180 559
- FR-A1- 2 877 222
- FR-A1- 2 940 059
- GALINSKI E A ET AL: "Microbial behaviour in salt-stressed ecosystems", DIVERSITY AND APPLICATIONS OF BACILLUS BACTERIOCINS, ELSEVIER, AMSTERDAM; NL, vol. 15, no. 2-3, 1 October 1994 (1994-10-01), pages 95-108, XP023708114, ISSN: 0168-6445, DOI: 10.1111/J.1574-6976.1994.TB00128.X [retrieved on 1994-10-01]
- FRINGS E ET AL: "Compatible solutes in representatives of the genera Brevibacterium and Corynebacterium: Occurrence of tetrahydropyrimidines and glutamine", FEMS MICROBIOLOGY LETTERS, NO LONGER PUBLISHED BY ELSEVIER, vol. 109, no. 1, 1 May 1993 (1993-05-01), pages 25-32, XP023853772, ISSN: 0378-1097, DOI: 10.1111/J.1574-6968.1993.TB06138.X [retrieved on 1993-05-01]
- LIPPERT K ET AL: "ENZYME STABILIZATION BY ECTOINE-TYPE COMPATIBLE SOLUTES: PROTECTION AGAINST HEATING, FREEZING AND DRYING", APPLIED MICROBIOLOGY AND BIOTECHNOLOGY, SPRINGER VERLAG, BERLIN, DE, vol. 37, no. 1, 1 April 1992 (1992-04-01), pages 61-65, XP000608489, ISSN: 0175-7598, DOI: 10.1007/BF00174204

## Description

### Field of the invention

The present invention relates to dermal cosmetic compositions having osmoprotecting activity.

### State of the art

Osmotic shock or osmotic stress is a sudden change in the solute concentration around a cell, causing a rapid change in the movement of water across its cell membrane. Under conditions of high concentrations of either salts, substrates or any solute in the supernatant, water is drawn out of the cells through osmosis. This also inhibits the transport of substrates and cofactors into the cell thus "shocking" the cell. Alternatively, at low concentrations of solutes, water enters the cell in large amounts, causing it to swell and either burst or undergo apoptosis.

All organisms have mechanisms to respond to osmotic shock, with sensors and signal transduction networks providing information to the cell about the osmolarity of its surroundings, these signals activate responses to deal with extreme conditions. Although single-celled organisms are more vulnerable to osmotic shock, since they are directly exposed to their environment, cells in large animals such as mammals still suffer from these stresses under some conditions.

Osmoprotectants are highly soluble organic molecules, electrically neutral at physiological pH, having the function to protect the cell against osmotic stress. Possible mechanisms have been proposed in the past decades, such as both those implying a direct interaction of the osmoprotectant with the protein and those based on an indirect effect of the osmoprotectant. Recently the most reliable theories encompass that osmoprotectant act with two different mechanisms the former stabilize the protein with an indirect pattern, based on the so called solvent structure forming and the latter based on the so called osmophobic effect.

In the first mechanism the osmoprotectant stabilize the microscopic structure of the solvent, by creating a network of hydrogen bonds with the water molecules. In addition osmoprotectants are able to reduce the auto diffusion coefficient of water, affecting therefore not only the structural but also the dynamic properties of water. The solvent thus disturbed results less prone to interact with the hydrophobic proteic residues, forming the central core of the protein, responsible for imparting stability to the same.

It follows that the folded protein conformation results indirectly stabilized.

Moreover it was observed that osmoprotectants in solution are not inclined to gather around the protein, rather they are excluded from the protein surface. This feature is the so called "osmophobic effect".

Thanks to the above properties the presence of these osmoprotectants in cosmetic formulation could have advantages especially in the treatment skin stress such as skin dryness.

FR 2 940 059 discloses skin hydrating compositions comprising at least two osmolitic agents chosen among taurine, inositol, betaine and trehalose. The Applicant has found that some osmoprotectants, when applied not to individual cells, but to a more complex whole of cells, i.e. a tissue like stressed skin, are unable to show the aforementioned properties.

Two compounds of this type are for example ectoine and trehalose, that, when applied alone to stressed skin are unable to show the aforementioned osmoprotecting properties.

The need is therefore felt to find substances, that also when applied to stressed skin are able to protect it against osmotic shock.

### Summary of the invention

The Applicant has now unexpectedly found that ectoine and trehalose, which as above pointed out when applied alone to stressed skin are inactive, when applied in association thereof on a cellular tissue, for example skin, are able to counter the effects caused by osmotic shock.

The present invention therefore relates to cosmetic compositions and in particular dermal cosmetic compositions comprising as the active ingredients an association of ectoine and trehalose, in total amounts ranging from 0.5 to 2% in combination with suitable excipients and/or diluents.

### Description of the figures

Fig. 1 reports the AQP3 Gene expression in non stressed but treated skin using as the calibrators the negative controls at 16 and 48 h (CN 16h=1, CN 48h =1) as described in the experimental part
FIG. 2 reports the AQP3 Gene expression in controls; Control in Standard Condition has been used as calibrator CN=1.
FIG.3 reports the AQP3 Gene expression in positive stressed controls and treated samples, wherein CONTROL RHE (Reconstructed Human Hepidermis)at 16 and 48h has been used as the calibrator CN=1.
Fig.4 reports the AQP3 Gene expression in stressed saline and treated tissues, wherein STRESSED SALINE is used as the calibrator CN=1
FIG.5 reports HSP1A1 Gene expression in non stressed but treated tissues; CN16h and CN48h have been used as calibrator.
FIG.6 reports HSP1A1 Gene expression in stressed and treated tissues, stressed saline has been used as calibrator.
FIG.7 reports the HSP1A1 Gene expression in stressed saline and treated tissues, where the stressed saline has been used as the calibrator CN=1.
Fig.8 reports the preferential coefficients of ectoine 1M and trehalose 1M.
Fig. 9 reports the preferential coefficients of ectoine 1 M and trehalose 1M in admixture.
Fig. 10 reports the preferential coefficients of ectoine 2 M and trehalose 2M.

### Detailed description of the invention.

The cosmetic composition according to the present invention contain ectoine and trehalose in molar ratios ranging from 1:5 to 5:1, preferably from 1:4 to 4:1, even more preferably from 1:3 to 3:1, According to a particular preferred embodiments these molar ratio range from 1:2 to 2:1.

In the cosmetic composition ectoine + trehalose total amounts range from 0,5 to 2% more preferably from 1 to 1,5% based on the total composition weight.

The osmoprotecting activity of the association of ectoine and trehalose at the aforementioned low dosages is quite surprising, also taking into account that the Applicant has demonstrated by carrying out a model simulation, reported in the experimental part of the present description that the osmoprotecting activity towards the C12 protein exhibited by the association of ectoine and trehalose is observed only in the presence of very concentrated solution containing respectively ectoine 1M and trehalose 1M, in other words with a total concentration of ectoine and trehalose 2M and that the best results are obtained in the presence of ectoine and trehalose respectively at concentration 0.75 M and 1.5 M and 1.5 Mm but also 1.5 and 0.75M amounting in both cases to a total equal to 2.25 M. Therefore at concentrations of at least two magnitude order higher than the highest concentration of these osmoprotectants in the cosmetic composition of the invention.

The cosmetic composition of the invention may comprise at least one further active ingredient selected from ceramide, in particular ceramide 3, cholesterol, fatty acids in particular stearic acid, vitamins in particular vitamin A and E, peptides, preferably oligopeptides, hyaluronic acid, Lysolecithins, lysophoshatidic acid, ferment lysates and mixtures thereof.

The cosmetic compositions of the invention may be formulated with conventional excipients used in the cosmetic field such as: Aqua, Glycerin, Caprylic/Capric triglyceride, Dimethicone, Aluminum starch octenylsuccinate, Butylene glycol, Tribehenin PEG-20 esters, Macadamia ternifolia seed oil, Dipentaerythrityl hexacaprylate/Hexacaprate, Cetyl alcohol, Olus oil, Cyclopentasiloxane, Dimethiconol, Hydrogenated lecithin, PEG-20 methyl glucose sesquistearate, Polysilicone-11, Butylene glycol dicaprylate/Dicaprate, Caprilyl methicone, Tapioca starch, Triethylhexanoin, Trimethylsiloxyphenyl dimethicone, Ammonium acryloyldimethyltaurate/Beheneth-25 Methacrylate Crosspolymer, Chlorphenesin, Disodium EDTA, Xanthan gum, C13-14 isoparaffin, Candelilla cera, Caprylyl glycol, Chlorphenesin, Glyceryl behenate, Glyceryl dibehenate, Hydrogenated vegetable oil, Bis-ethylhexyl hydroxydimethoxy benzylmalonate, Laureth-7, Mannitol, Mica, Parfum, Phenoxyethanol, Dibutyl adipate, Dicaprylyl carbonate, Bis-ethylhexyloxyphenol methoxyphenyl triazine, Methylene bis-benzotriazolyl tetramethylbutylphenol, Ethylhexyl salicylate, Ethylhexyl triazone, Butyl methoxydibenzoylmethane, Octocrylene, Glycerin, Cocoglycerides, Diethylamino hydroxybenzoyl hexyl benzoate, Acrylates/C10-30 alkyl acrylate crosspolymer, Decyl glucoside, Polyacrylamide, Potassium sorbate, Sodium dehydroacetate, Sodium hydroxide, Sodium phytate, Sodium stearoyl lactylate, Stearic acid, Tetrasodium EDTA, Tribehenin, Ammonium acryloyldimethyltaurate/Beheneth-25 Methacrylate Crosspolymer, Triethoxycaprylylsilane, Citric acid, Alcohol, pigments based on iron oxides such as CI 77491, CI 77891.

These cosmetic compositions are in particular in the form of w/o or o/w emulsions, sera ointment, gel-creams, body milks, sun and after sun milks, sun and after sun gel-cream and sun sera etc.

As pointed out above the cosmetic compositions of the invention and unlike those containing respectively only ectoine and trehalose, exhibit osmoprotecting property against skin osmotic stress as demonstrated by the following experimental tests herewith reported only for illustrative purposes.

### EXAMPLE 1

An experimental model of epidermal dryness, induced by modified room humidity and temperature conditions, has been used to assess the efficacy of 3 cosmetic products characterized by the following compositions
SOLUTION 1 Water + trehalose 1%
SOLUTION 2 Water + ectoine 0,5%
SOLUTION 3 Water + ectoine 0,5% + trehalose 1%.

### 1 MATERIALS

### 1.1.TEST SYSTEM

### Human reconstituted Epidermis

SkinEthic^{®} Reconstructed Human Epidermis (RHE) of 0,5 cm² is a fully differentiated epidermis formed after 17 day of air-lift culture of NHK from skin biopsies in a chemically defined medium. The model reproduces epidermal morphology and it has been fully characterized. The good barrier function, batch reproducibility and low variability in terms of permeability for probe molecules have been described in the Literature compared to human ex-vivo models.

| NAME and BATCH | Reconstituted Human Epidermis (RHE) 12 022A 0211 | |
|---|---|---|
| MANUFACTURER | SKINETHIC (F) | |
| HISTOLOGY AT DAY 17 Number of cell layers (>4) | 5/6 | Accepted |
| CELL VIABILITY AT DAY 17 (>0,7) | 1,521 ± 0,083 | Accepted |
| DATE OF ARRIVAL | 13.03.2012 | |
| EXPIRATION DATE | 19.03.2012 | |

### 1.1.1 MEDIA, CULTURE CONDITION AND PREPARATION OF THE CULTURE

The tissue and media are manufactured in compliance with ISO 9001. Immediately after arrival in the laboratory, the RHE were transferred to a 6-well plate previously filled with maintenance medium (1 mL/well) at room temperature and incubated at 37°C, 5% CO₂, saturated humidity. The test has been started the day after the arrival.

### 1.2. TEST ITEMS: : IDENTIFICATION AND CHARACTERIZATION

The above mentioned 3 water solutions have been stored and dosed at the following operating conditions

| NAME | SOLUTION 1 | SOLUTION 2 | SOLUTION 3 |
|---|---|---|---|
| UNIVOCAL CODE | SOL 1 | SOL 2 | SOL 3 |
| STORAGE | 2-8°C | 2-8°C | 2-8°C |
| CONCENTRATION OF USE | Pure | Pure | Pure |
| DOSE | 50 µL | 50 µL | 50 µL |

### 1.3 NEGATIVE CONTROL AND REFERENCE PRODUCT

NEGATIVE CONTROL was the saline solution (0,9% NaCl) which is characterized by its neutral action on tissues.

### 2. METHODS

### 2.1 LDH QUANTIFICATION

The cell membrane forms a functional barrier around the cell, and traffic into and out of the cell is highly regulated by transporters, receptors and secretion pathways. When cells are damaged, they become 'leaky' and this forms the basis for the second type of assay. Membrane integrity is determined by measuring lactate dehydrogenase (LDH) in the extracellular medium. This enzyme is normally present in the cytosol, and cannot be measured extracellularly unless cell damage has occurred. A commercially available kit (Cytotoxicity Detection KIT-LDH, Roche) has been used to quantify the LDH released in culture media by a colorimetric assay based on formazan salt detection (λ 492 nm with reference at 690nm). The culture supernatant is collected and incubated with the reaction mixture including the kit (20 min, room T°, in the dark). An increase in the amount of dead or plasma membrane-damaged cells results in an increase of the LDH enzyme activity in the culture media. This increase in the amount of enzyme activity in the supernatant directly correlates to the amount of formazan formed during a defined time period therefore, the amount of color formed in the assay is proportional to the number of lysed cells.

A standard curve using different concentrations of LDH: 125 mU/mL; 62,5; 31,25; 15,625; 7,8125 mU/ml has been previously determined.

### 2.2 REAL TIME PCR

### 2.2.1 RNA extraction, cDNA Retrotranscription and REAL TIME PCR

For the three steps, ready to use reagents were used. The RNAqueous method is a rapid, phenol free, filter based RNA isolation system used to extract the total RNA from cellular samples. The High Capacity cDNA Reverse Transcription kit was used to synthetize cDNA from RNA. The instrument Applied Biosystems 7500 Fast Real Time PCR with fluorescent-based PCR chemistry, the TaqMan assay, was used to study gene expression of significant biomarkers. Gene expression is the process by which the inheritable information in a gene, such as the DNA sequence, is made into a functional gene product, such as protein or RNA. Relative quantification determines the change in the expression of a nucleic acid sequence in a test sample relative to the same sequence in a calibrator sample. Beta-actin was used as an endogenous control gene to normalize input amounts.

Each replicate was assessed in triplicate. At the 2X TaqMan Fast Universal PCR Master Mix was added Taqman gene expression assay and cDNA (25 ng) for a total volume of 25 µL. The Thermal condition steps in the ABI PRISM 7500 Fast are: 95°C 20 sec; 40 cycles (95°C 3 sec +60°C 30 sec).

### 2.2.2 DATA ACQUISITION AND ACCEPTANCE CRITERIA

Fluorescence data of the RT-PCR generated by the thermocycler ABI PRISM 7500 Fast, are collected by the internal software SDS 2.0.5 and the Raw Data (RQ study results) are printed and exported in Excel.

(These data contained the following information: Biological group, Target, Omitted, Technical replicates, RQ, RQ Min, RQ Max). Because each cycle in the PCR reaction corresponds to a 2-fold increase in PCR product, a difference of one in threshold cycle number represents a 2-fold difference in the expression of a particular gene compared to the calibrator sample and can be considered as significant. 95% of confidence level is used by the software to calculate the errors.

### 3. STATISTICAL ANALYSIS

No specific statistical analysis has been carried out except the Standard Deviation calculation.

### 4. RESULTS

### 4.1 LDH QUANTIFICATION

The LDH release results, expressed as % of citotoxicity are reported in Tab. I

**TAB. I LDH release expressed as % of citotoxicity**

| TABLE I | % VIABILITY |
|---|---|
| CONTROL 16h | 91,55 |
| STRESSED RHE 30 MIN + 16H | 0 |
| STRESSED + SALINA 30 MIN + 16H | 52,06 |
| GLY 5% 30 MIN + 16H | 80,70 |
| SOL 1 30 MIN + 16H | 76,82 |
| SOL 2 30 MIN + 16H | 48,01 |
| SOL 3 30 MIN + 16H | 53,15 |
| SOL 1 STANDARD 30 MIN + 16H | 83,44 |
| SOL 2 STANDARD 30 MIN + 16H | 92,34 |
| SOL 3 STANDARD 30 MIN + 16H | 99, 95 |
| GLY 5% STANDARD 30 MIN + 16H | 96,08 |
| | |
| CONTROL 48H | 79,08 |
| STRESSED RHE 30 MIN + 48H | 80,23 |
| STRESSED + SALINA 30 MIN + 48H | 69,27 |
| GLY 5% 30 MIN + 48H | 61,11 |
| SOL 1 30 MIN + 48H | 56,18 |
| SOL 2 30 MIN + 48H | 62,63 |
| SOL 3 30 MIN + 48H | 34,56 |
| SOL 1 STANDARD 30 MIN + 48H | 71,46 |
| SOL 2 STANDARD 30 MIN + 48H | 93,26 |
| SOL 3 STANDARD 30 MIN + 48H | 90,33 |
| GLY 5% STANDARD 30 MIN + 48H | 63,14 |

The higher optical density has been quantified in the media of **stressed RHE** and used as 100 % higher citotoxicity value. The optical density of Negative Control has been considered as a control lower value.

In the media of **Control tissues (16h-48h**) any citotoxicity has not been quantified (91,55 % and 79% viability respectively).

**STRESSED RHE** was found as the high control stressed sample after 16h recovery (100 % citotoxicity) but it recovered the viability after 48h (80,23 %).

Stressed **RHE treated with stalin solution** has shown a similar behaviour in short term and long term recovery (52,06 and 69,27%).

All the products applied in standard conditions (without stress) and let for 16h and 48h were not citotoxic. Glycerin 5% resulted not citotoxic either in stressed conditions after 16h or 48h recovery or in standard conditions.

The only significant peak of citotoxicity was found after 48h recovery of tissues treated with SOL3 (34,56 % viability).

It is important to underline that a high variability has been found between the two biological series of each treatment (Appendix II).

In the paragraph 6.1, LDH was expressed as the mean of duplicates, but these data were not used to classify the products efficacy.

**TEER (Trans epithelial electric resistance)** has been measured on all the tissues arrived, at basal level: after 30' stress induction at -20°C the TEER was severely dropped from 10.000 Ω*cm² to values under 500 Ω*cm² associated to a severe modification of paracellular flux. No difference in TEER measurements were found after 16h and 48h of recovery. All the tissues had a value of around 10000 Ω*cm² and these data were confirmed at all time points.

### 4.2 REAL TIME PCR RESULTS

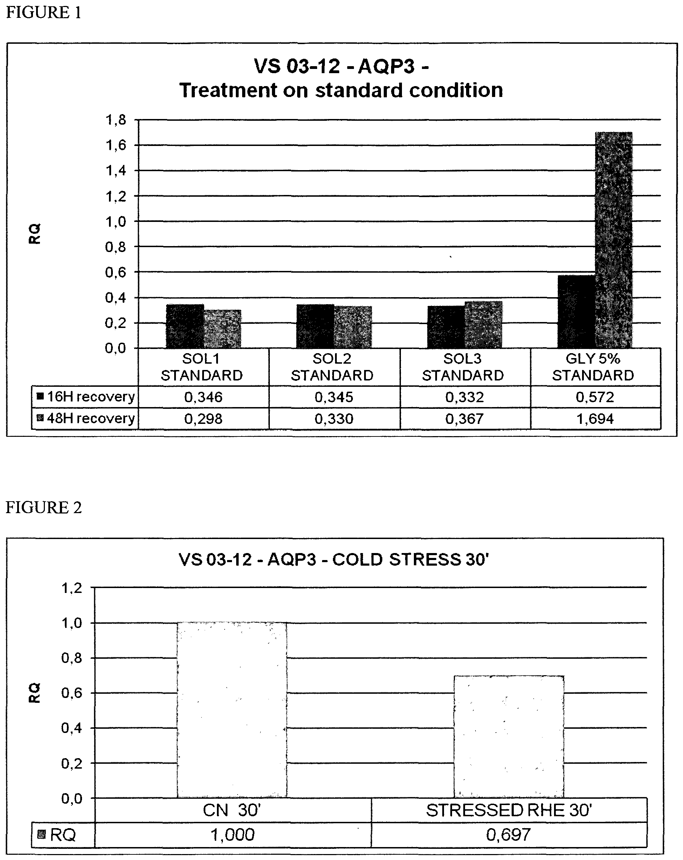

In the Fig.1 the **TARGET GENE AQP3** was analyzed using the negative controls as the calibrator samples (CN 16h =1 and CN 48h =1).

SOL1, SOL2, SOL3 and GLYCERIN 5% have been applied on the epidermal surface of cold stressed RHE for 16h and 48h treatment.

Glycerin 5% has significantly reduced the expression of AQP3 after 16h treatment wherease in the late recovery time, 48h, has induced a( although not significant) over-expression of AQP3 (RQ= 1,694) that is correlated to the activation of water channels.

The 3 solutions (SOL1, SOL2 and SOL3) have significantly reduced the expression of AQP3 after incubation for 16h or 48h in standard conditions.

These results suggest that in absence of stress the 3 solutions have determined a block of water flux resulting in a strong conservative pathway of water content.

Figure 2 reports the AQP3 Gene expression in controls; Control in Standard Condition has been used as calibrator CN=1.

After 30 minutes of cold induction, the stressed RHE reduced AQP3 gene expression (RQ=0,697) (Fig. 2).

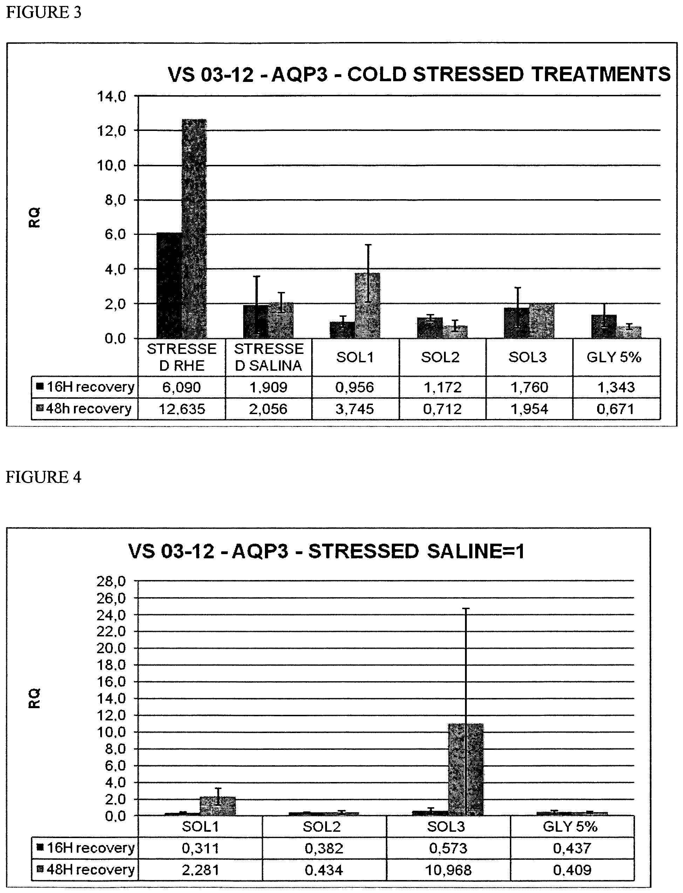

The cold stress performed by 30' induction at -20°C corresponds to a block of water flux: the treatment has been done at this moment and followed by 16h and 48h recovery allowing to monitor the dynamic of the genomic response and to study product mechanism of action: the fig.3 described RHE's physiological response of saline solution treatment and glycerol compared to the RHE physiological response of the same treated with SOLUTIONS 1,2,3.

After 16h and 48h recovery, the stressed RHE increased the levels of AQP3 gene (RQ=6,090 and 12,635 respectively) as expected a defence mechanism has been established in order to restore water flux.

SOL1, applied after the 30' stress induction, induced an over-expression of AQP3 after 48h of recovery (RQ=3,745). SOL3 increased near the significance values the expression of AQP3 after incubation of 48h of recovery (RQ=1,954).

After 48h recovery, both SOL2 and Glycerin 5% reduced the expression of AQP3.

Since the RHE stressed saline solution resulted to have an influence on the AQP-3 expression (RQ=2, significant up-regulation) and also because it represented the placebo in this experimental set-up applied better than the simple stressed RHE, the positive stress was used as the calibrator and settled =1 for a better definition of the efficacy of the products (Fig. 4).

SOL1 and with higher values SOL3 have induced a significant over-expression of AQP3 after 48h of recovery (RQ=3,745) in comparison to RHE STRESSED SALINE used as calibrator. SOL2, like Glycerol 5%, reduced the expression of AQP3.

The high standard deviation reflects the difference in LDH release of the two biological replicates.

It is interesting to interpret the results not only in term AQP3 expression but also in term of kinetics of product's mechanism in the cold stress model:
- the reference glycerol was inactive in restoring the physiological water flux and hydration after stress, thus determining an active moisturizing mechanism at molecular level, confirming its principal mechanism of action as hygroscopic molecule. Glycerol in not stressed RHE has determined an over expression of AQP3 in the long term recovery (fig.1) suggesting again a direct influence on intracellular water.
- the 3 solutions act first, in the early recovery time (16h) by a conservative pathway of water flux where the AQP-3 gene expression has been blocked allowing the cells to keep the physiological water content as it has been observed when the actives were applied in non stress conditions: the observed protective efficacy of water cluster binding mechanism has been confirmed.
- the kinetics of an active hydration mechanism at molecular level is clearly shown by the 48h results where the over expression of AQP-3 finally determines the opening of water channel and enhances the water income and moisturizing efficacy. This mechanism has been confirmed by SOL1 and 3 and not for SOL 2.

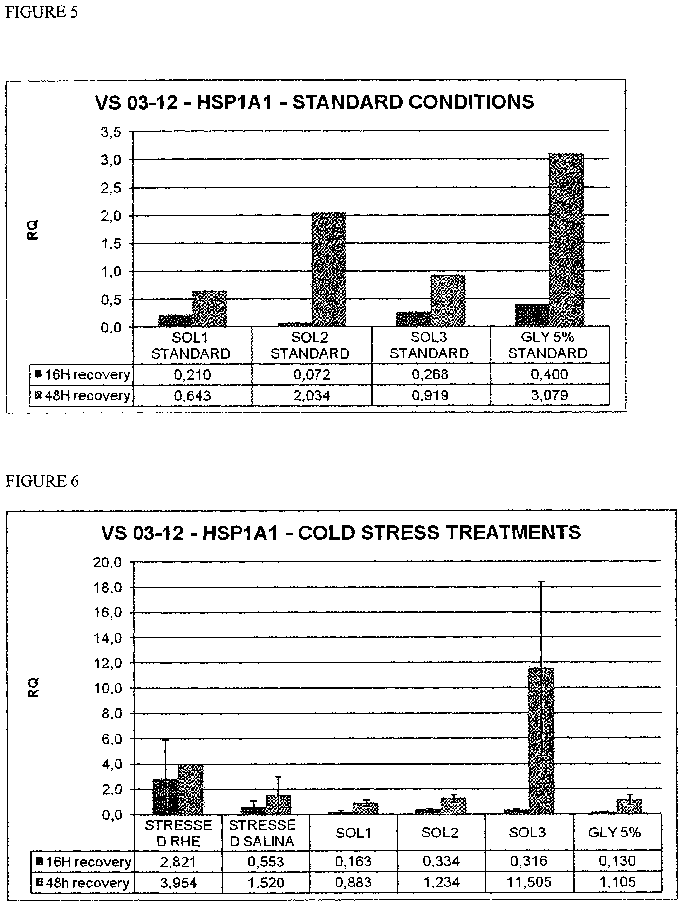

Fig.5 the TARGET GENE HSP1A1 was analyzed using the negative control as calibrator samples (CN 16h =1 and CN 48h =1).

Again it is interesting to discuss not only the expression levels but also the dynamic of the genomic response :
The 3 solutions (SOL1, SOL2 and SOL3) and Glycerin 5% have significantly reduced the expression of HSP1A1 after incubation for 16h in standard condition.

In the late recovery time, 48h treatment, an over expression of HSP 72 has been quantified only for SOL2 and Glycerin 5% : SOL 1 and SOL 3 did not significantly modulate the gene when applied in not stressed RHE.

In the Fig.6 TARGET GENE HSP1A1 was analyzed using the positive stressed control as calibrator samples (CN 16h =1 and CN 48h =1).

After 16h and 48h recovery in the stressed RHE the levels of HSP 72 gene (RQ=2,821 and 3,954 respectively) increased.

Since the saline solution treatment resulted to have an influence on the HSP72 expression (down regulation) and also because it better represented the placebo in this experimental set-up applied in comparison with stressed RHE, the positive stress was used as calibrator and settled =1 for a better understanding of SOL-1,2 and 3 efficacy (fig. 6).

SOL1, SOL2, SOL3 and GLYCERIN 5% have reduced the expression of HSP 72 after 16h of recovery: as observed for AQP3 where a conservative pathway of water was established and an adaptive mechanism also for HSP 72 is observed at this time.

After 48h recovery, only SOL3 significantly increased the expression of HSP 72.

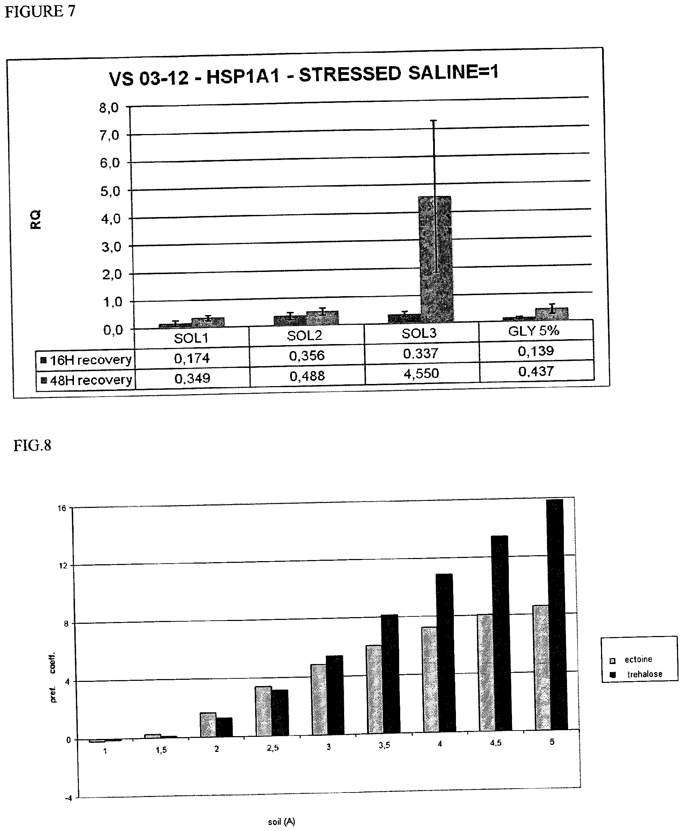

Figure 7 TARGET GENE HSP1A1 was analyzed using the stressed saline solution(CN 16h =1 and CN 48h =1).

A down-regulation of HSP 72 after 16h of recovery in RHEs treated with SOL1, SOL2, SOL3 and GLYCERIN 5% was quantified, on the contrary a different kinetics was observed for SOL3 that in the late recovery period increased the expression of HSP 72.

### 5. DISCUSSION

In order to understand the mechanism of action on water binding and epidermal water's cluster protection compared to their components **(ECTOIN** and **THREALOSE)** and to **GLYCEROL as reference hygroscopic product, a** modified in vitro dryness model has been proposed based on a short but severe cold stress followed by early and late recovery periods modelled in order to follow the dynamics of the genomic response driven by active's efficacy.

The 3 products named as **SOL 1, SOL 2, SOL 3** have also been applied in standard conditions in order to extrapolate their direct influence in non stress conditions.

Osmotic stress has been induced by **cold conditions** (-20 °C) for **30 minutes:** TEER was severely reduced and the damage at membrane level has been confirmed by LDH release.

The cold stress model described had been followed by a physiological defence response (16h and 48h recovery) allowing to monitor the dynamic of the genomic response (AQP3 and HSP1A1) and to study product's mechanism of action.

The main results are summarized herein:
**COLD STRESS:** RHE stressed for 30 minutes at -20°C
   ➢ reduced AQP3 gene expression (RQ=0,697) (Fig. 2)
   ➢ no modulation of HSP1A1 gene expression has been observed(Fig. 6)
**RECOVERY PERIODS: tissue physiological defence response RHE STRESSED** placed in standard conditions for 16h and 48h recovery
   ➢ 16h recovery: increased AQP3 gene expression increased HSP1A1 gene expression
   ➢ 48h recovery: increased AQP3 gene expression increased HSP1A1 gene expression
**RHE STRESSED SALINE,** treated with saline solution, placed in standard conditions for 16h and 48h recovery
   ➢ 16h recovery: increased AQP3 gene expression (less than RHE stressed alone), downregulation of HSP1A1 gene expression
   ➢ 48h recovery: increased AQP3 gene expression (less than RHE stressed alone)not significant modulation of HSP1A1 gene expression.

RHE STRESSED and treated with SALINE was used as calibrator control of cold stress induction, because it better represents a stressed tissue in which saline solution has the role of placebo for the 3 solutions.

### COLD STRESS MODEL RATIONEL

The cold stress induces an entrapment of water in the tissues, forming inactive water clusters, that can be modified, activated by product's efficacy.

Based on the results of negative and positive control and placebo (saline treated RHEs) and in order to define osmoprotecting activity, a product applied after the cold stress induction and let in standard conditions for 16h and 48h, should satisfy the following requirements
- **increasing AQP3 gene expression**
- **up-regulating HSP 72 gene expression** (cytoprotective and anti-inflammatory effect)

### AQP-3

AQPs are proteins that facilitate the transport of water across cell membranes.

AQP-3 in particular has a key role in the transport and distribution of epidermal water and glycerol and the regulation of keratinocyte differentiation, both critical processes for maintaining skin hydration, barrier function and overall skin health.

The absence of AQP3 in mammalian skin resulted in skin dryness, reduced stratum corneum hydration, reduced skin elasticity, and delayed barrier recovery.

The defective osmotic equilibrium that could occur in the epidermis and that would account for the skin dryness strongly affected the expression of AQP-3 water channels. The increase of AQP-3 gene allows cells to keep the physiological water content corresponding to a protective efficacy.

SOL1, SOL2, SOL3 applied on the epidermal surface of RHE for 16h and 48h treatment have been significantly reduced the expression of AQP3 in standard conditions suggesting a block of water channels and conservative water flux. SOL1 and SOL3, applied after stress induction induced an over-expression of AQP3 after 48h of recovery showing a protective conservative pathway of water cluster in the early read-out and a moisturizing effect in the late recovery period.

SOL2, like Glycerin 5%, did not significantly modulate the expression of AQP3.

Glycerol hygroscopic mechanism had been indirectly confirmed by these results: hygroscopic molecules have not an active mechanism on epidermal hydration; in environmental stress conditions they adsorb water from the tissue resulting in a dehydration mechanism.

The human HSP70 family consists of at least 12 members (Tavaria *et al* 1996):
- the constitutively expressed HSP70 (Hsc70 or HSP73)
- the stress inducible HSP70 (HSP70 or HSP72) with the fundamental role to protect cells against cellular stress.
- the mitochondrial HSP70 (HSP75)
- the endoplasmic reticulum HSP70 (Grp78).

Heat shock proteins (HSP) were originally described for their roles as chaperones induced by temperature shock as well as various other kinds of stress including environmental (UV radiation, heat shock, heavy metals and amino acids), pathological (bacterial, parasitic infections or fever, inflammation, malignancy or autoimmunity) or physiological stresses (growth factors, cell differentiation, hormonal stimulation or tissue development), that induced a marked increase in intracellular HSP (iHSP) synthesis known as the stress response (Park HS et al. 2002).

Recently an additional role has been ascribed to HSP as danger signals produced and released when cells are under stress and as activators of the immune system.

The up-regulation of intracellular HSP70 (iHSP72) has a cytoprotective effect and furthermore induces the cell's anti-apoptotic mechanisms, modulates cell cycle progression and has an anti- inflammatory action.

The 3 solutions (SOL1, SOL2 and SOL3) and Glycerin 5% reduced the expression of HSP 72 after incubation of 16h in standard conditions as expected because there was no need to protect cells.

However SOL2 and Glycerin 5% has significantly increased the expression of HSP 72 after 48h treatment, showing a direct cytoprotective effect.

SOL1, SOL2, SOL3 and GLYCERIN 5% applied after the stress induction, have significantly reduced the expression of HSP 72 either after 16h and 48h of recovery offering a poor cell protection.

SOL3 significantly increased the expression of HSP1A1 after incubation of 48h of recovery showing cytoprotection and anti-inflammatory response.

The results reported in the following table summarize the results reported for each solutions 1-3 of the experiments carried out and described previously

**OSMOPROTECTING EFFECT: EFFICACY EVALUATION**

| 16H/48H RECOVERY | AQP3 (RQ) | HSP 72 (RQ) | "OSMOSHIELD EFFICACY" |
|---|---|---|---|
| SOL1 | 0,311 / 2,281 | 0,174/ 0,349 | NO |
| SOL2 | 0,382 / 0,434 | 0,356 / 0,488 | NO |
| SOL3 | 0,573 / 10,968 | 0,337 / 4,550 | YES |
| GLYCERIN 5% | 0,437 / 0,409 | 0,139 / 0,437 | NO |

Only SOLUTION 3 has an osmoprotecting long lasting hydration efficacy (48h after the stress) on the induced epidermal cold stress and an early conservative pathway of water cluster.

The product enables the release of water from water clustered during the cold stressed tissue (-20°C) thanks to the AQP 3 over-expression and a cyto-protective conservative pathway of protein's structure on the basis of the HSP-72 overexpression at the time 48h.

### EXAMPLE 2 RANDOMIZED BLIND TEST

We report herewith a clinical test carried on 20 female volunteers characterized by dry and sensitive face skin, treated in one part of the face for 3 weeks with a gel, defined "active gel" characterized by the following composition:

| ACTIVE GEL | |
|---|---|
| Aqua | 96.400 |
| Sodium polyacrylate | 1.500 |
| Trehalose | 1.000 |
| Citric acid | 0.400 |
| Ectoin | 0.500 |
| Sodium dehydroacetate | 0.200 |

and at the other part with the composition defined

| PLACEBO GEL | |
|---|---|
| Aqua | 97.900 |
| Sodium polyacrylate | 1.500 |
| Citric acid | 0.400 |
| Sodium dehydroacetate | 0.200 |

for a total of 3 weeks.

### 2.1 OBJECTIVE OF THE TEST

Purpose of the test is to evaluate the effect on the cutaneous microcirculation flow and the cutaneous density.

### 2.2 ADMISSION AND RECRUITING CRITERIA

The test was carried out on 20 female volunteers having dry and sensitive face skin of average age: 49.7 years. The selection was carried out according to the following criteria.
- Caucasian race;
- Women being 30-60 years old in good health and having dry skin face;
- Subjects able to follow the tests directives and present themselves at every control for the entire duration of the test.
- Subjects having completed the information process and signed the written consent
- Subjects accepting not to expose themselves to the sun or to UV lamp for the entire duration of the test.
   and exclusion criteria:
- Pregnant or breast feeding women,
- Subject with previous cutaneous hypersensitivity episodes to cosmetic products or being sensitive to any component of the product to be tested.
- Subject in course of systemic or topic treatment with any drug that may interfere with the test results.
- Subjects with systemic pathologies or cutaneous disorders (such as eczema, psoriasis, severe acne, non homogenous skin colour etc.), that may interfere with the test results or increase the risk for the volunteer.
- Subjects to anti wrinkle (topic or systemic) treatment;
- Subject under other clinical tests or who participated to clinical tests within 30 days prior to the present test.

### 2.3 DROP OUT

The following reasons are considered causes of interruption of the test:
- Subjects' free choice;
- Medical reasons non correlated to the treatment (diseases onset, surgical operations)
- Medical reasons correlated to the treatment (irritating or allergic reactions)

### 2.4 RESTRICTIONS

During the test the volunteers are asked to use for daily cleaning the usual detergent.

For the entire duration of the test subjects are not permitted to apply different products on the zone undergoing the test and should avoid UV exposure of the same area.

### 2.5 INSTRUMENTS

The following instruments have been used for carrying out the test:

### Flowmeter periflux PF4001, Perimed

This instrument measures the rate and the volume of surface hematic capillary flow of skin.

### Ultrasound Scanner Dermascan C^{®}

This instrument is a high resolution ecograph scanner with HF ultrasounds emission (20MHz, allowing to study skin up to 15mm depth with an axial resolution of 60µm and side resolution of 200µm.

This instruments measures the derma density and its main components. Young skin exhibits a higher density if compared to mature skin.

The effectiveness of a densifying results in a significant increase in skin density, denoting an improvement in dermal structure.

### 2.6 METHOD

The instruments measurements were carried out inside a room with controlled temperatures and humidity (24±2°C; 50±10% r.h.)

The volunteers were asked not to clean face 3 hours before carrying out the instruments measurements, and to apply no product on face 12 hours before carrying out said measurements.

At the trial onset the instruments measurements were carried out of cutaneous microcirculation blood flow and cutaneous density.

A second set of instrument measurements was carried out 2 hours after the application of the active gel and placebo gel (dose 2mg/cm²) on each half face of the volunteers. The application site (right or left) was randomized among the volunteers.

The products (active gel and placebo gel) are thereafter given to the volunteers that applied them with the same operative modalities above reported twice a day for 3 weeks.

At the end of the use period, the volunteers underwent the final instruments measurements.

For each measurements series the average values the standard deviation and variance was calculated.

On the base of the results of the normality tests (Kolgomonov- Smirnov's test, Lilliefors' test and Shapiro- Wilk' s test), the instrumental data (T0, T2h and T3weeks) were compared statistically by means of the variance analysis and Bonferroni's test for dependent data.

The data were considered statistically different for a probability value<= 0.05

A volunteer interrupted the test for reasons correlated to the product due to the appearance of side effect such as skin dryness and desquamation.

The results refer therefore to 19 volunteers.

### 2.6 RESULTS

2.6.1 Cutaneous microcirculation blood flow The results of this test are reported in the following table

**Table: average, standard deviation, variation. %variation and statistical test**

| | T₀ Average *Std. dev.* | T₂ₕ Average *Std. dev.* | T_{3wks} Average *Std. dev.* | Variation T₂ₕ- T₀ (% Variation) Bonferroni's Test | Variation T_{3wks} - T₀ (% Variation) Bonferroni's Test |
|---|---|---|---|---|---|
| Active gel | 62.37 | 52.14 | 52.35 | -10.23 | -10.02 |
| | *26.83* | *21.49* | *13.36* | (-16.4) | (-16.1%) |
| | | | | **p < 0.05** | **p < 0.05** |
| Placebo gel | 62.41 | 56.99 | 63.06 | -5.42 | +0.65 |
| | *26.76* | *21.14* | *21.08* | (-8.7) | (+1) |
| | | | | p>0.05 | p>0.05 |

The treatment with active gel resulted in a statistically significant decrease of the basal values of cutaneous microcirculation blood flow both after 2 hours from the application and after 3 weeks of treatments. No statistically significant variations were observed following the treatment of placebo.

In addition as it results from the table herein below reported, no statistically significant difference was observed between the Active Gel and the Placebo gel. Table Statistical comparison between Active Gel and Placebo gel.

| | Bonferroni's Test T₂ₕ- T₀ | Bonferroni's Test T_{3wks} - T₀ |
|---|---|---|
| Active gel vs. Placebo gel | p>0.05 | p>0.05 |

### 2.6.2. Cutaneous density/ecogenicity

The results of this test are reported in the following table

**Table: average, standard deviation, variation. %variation and statistical test**

| | T₀ Average *Std.dev.* | T₂ₕ Average *Std. dev.* | T_{3wks} Average *Std.dev.* | Variation T₂ₕ- T₀ Bonferroni's Test | Variation T_{3wks} - T₀ Bonferroni's Test |
|---|---|---|---|---|---|
| Active gel | 22.74 | 22.95 | 27.24 | +0.21 | + 4.50 |
| | 4. 72 | 5.40 | *3.46* | p>0.05 | **p < 0.05** |
| Placebo gel | 24.34 | 24.74 | 26.27 | +0.40 | +1.93 |
| | *6.11* | 6.27 | *5.18* | p>0.05 | p>0.05 |

2 hours after the application of both products no significant variation was observed in the basal values of cutaneous density.

The repeating application of the active gel induced a statistically significant increase of cutaneous density for the active gel, whereas the variation for the placebo gel was not statistically significant.

### 2.7 Conclusions

The application of the active gel resulted in a statistically significant reduction of the basal cutaneous microcirculation blood flow towards the parameter normalization both after 2h and after 3 weeks of treatment.

The activity of active gel was moreover confirmed by the fact that no statistically significant difference was observed with placebo.

After 3 weeks of application the active gel brought to a statistically significant increase in cutaneous density, resulting in a densifying action of the active gel.

### EXAMPLE 3 SIMULATION TESTS

Applicant also carried out simulation of molecular dynamics using as the model the protein C12 in ectoine and/or trehalose solution to study the distribution of these compounds around the protein and evaluate a possible osmophobic effect.

### METHOD FOR CARRYING OUT DYNAMICS SIMULATION

The starting structure of the protein C12 was obtained by the data base PDB (CODE 1YPC). In all cases the protein was inserted in a virtual cubic box of 80 Å side, the necessary amount of osmoprotectas was added to reach the desired concentration. The osmoprotectant are inserted in casual positions inside the solvation box, thereafter the system was solvated with water described by the model TIP4P. Then, the protein being positively charged, the electro-neutrality of the system was guaranteed by the addition of two chloride anions.

The simulations were carried out using the software GROMACS 4.5.3. The system prepared as described above was subjected to 10000 optimization steps of geometry using alternately the steepest descendent (SD) and the conjugated gradient (CG) methods, in particular 1 SD step for 10 CG steps. The system was then balanced for 100 steps at constant volume and temperatures, followed by further 100 steps at constant pressure and temperature. During the equilibration phase the C_{α} atoms of the protein were fixed at their original position by means of an armonic bond. The equilibration was followed by a productive phase lasting 20 ns under constant pressure and temperature, wherein the armonic bonds on C_{α} were removed.

The temperature was maintained at the reference value (310°K) recalculating the particles rates forming the system , while maintaining the pressure at an average value of 1 bar coupling the system by isotropy to a baric bath using Berendsen's algorithm. The bond distances among all atoms were maintained at their equilibrium value using LINKS algorithm, allowing therefore to use an integration step of 2 fs *fs=femtosecondi.* All simulations were carried out using periodical boundary conditions, the electrostatic interactions were determined by means of Particle Mesh Ewald method, whereas in so far as the Van der Waals interactions are concerned, a cut-off of 14A was applied.

AMBER03 strength field was used to describe the protein, whereas GAFF strength field was used to describe osmoprotectants. The simulation analyses were carried out with GROMACS modules.

To evaluate the osmophobic effect, the preferential coefficients of the individual compounds (ectoine and trehalose) with respect to the protein was calculated. A positive preferential coefficient indicates a gathering of the compound (ectoine or trehalose)with respect to the solution bulk, a negative coefficient means a rarefying thereof.

In figure 8 the preferential coefficients of ectoine in 1M solution and trehalose in 1M solution are reported, as a function of the distance from the proteic surface. It is evident if we exclude the lower soil of 1 Å that for both compounds showing the aforementioned positive parameters no osmophobic effect can be observed at the aforementioned concentration since, both the preferential coefficient of ectoine and those of trehalose are positive and increasing with the distance from the proteic surface.

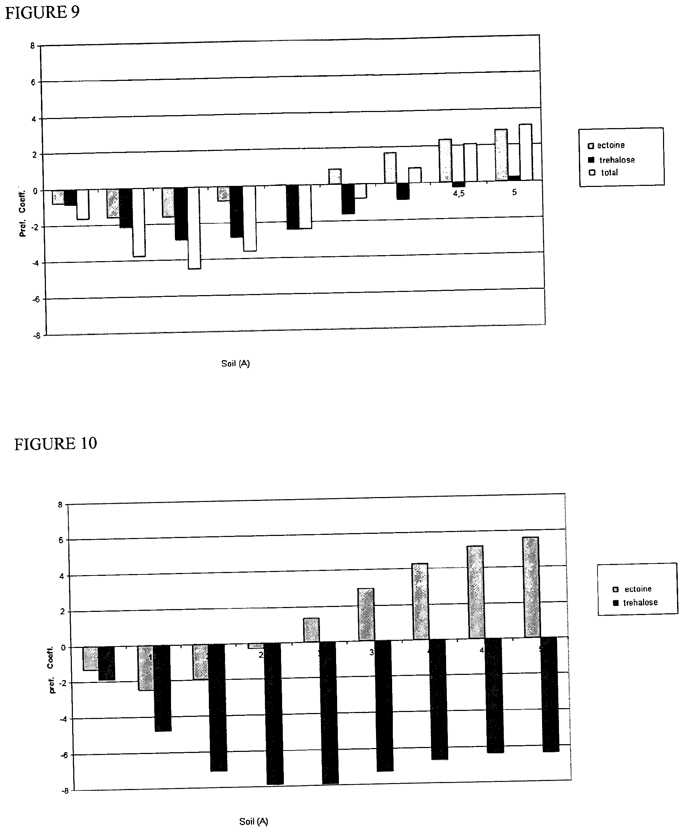

As reported in Fig. 9 a solution of ectoine and trehalose each at concentration 1 M gives the following results: it is osmophobic up to the soil of 2.5-3.0Å On the contrary ectoine and trehalose separately show osmophobic effect when they have 2M concentrations as is evident in Figure 10 wherein trehalose results to be always osmophobic, and ectoine results osmophobic up to 2.5Å.

It is evident therefore that the osmophobic feature is present when the total concentration of ectoine and trehalose either alone or the total concentration thereof, when they are in admixture is = 2M.

On the contrary the Applicant with the in vitro experimental test described before has surprisingly found that the mixture of trehalose and ectoine is active at total concentration which are of at least 2 magnitude lower than the lower concentration above indicate (2M), at which according to the simulation above reported the association of these osmoprotectants show osmophobic effect, by applying simulation models.

We report herewith for illustrative purposes some example of the cosmetic composition of the invention.

**Serum 1**

| | |
|---|---|
| Aqua | 54,358 ÷ 62,039 |
| Glycerin | 6,129 ÷ 6,995 |
| Caprylic/Capric triglyceride | 3,680 ÷ 4,200 |
| Dimethicone | 3,680 ÷ 4,200 |
| Aluminum starch octenylsuccinate | 2,760 ÷ 3,150 |
| Butylene glycol | 2,760 ÷ 3,150 |
| Tribehenin PEG-20 ester | 2,760 ÷ 3,150 |
| Macadamia ternifolia seed oil | 1,840 ÷ 2,100 |
| Dipentaerythrityl hexacaprylate/Hexacaprate | 1,380 ÷ 1,575 |
| Cetyl alcohol | 1,104 ÷ 1,260 |
| Olus oil | 1,104 ÷ 1,260 |
| Cyclopentasiloxane | 0,920 ÷ 1,050 |
| Dimethiconol | 0,920 ÷ 1,050 |
| Hydrogenated lecithin | 0,920 ÷ 1,050 |
| PEG-20 methyl glucose sesquistearate | 0,920 ÷ 1,050 |
| Trehalose | 0,920 ÷ 1,050 |
| Ammonium acryloyldimethyltaurate/Beheneth-25 Methacrylate Crosspolymer | 0,552 ÷ 0,630 |
| Glyceryl dibehenate | 0,552 ÷ 0,630 |
| Ectoin | 0,465 ÷ 0,530 |
| Bis-Ethylhexyl Hydroxydimethoxy benzylmalonate | 0,460 ÷ 0,525 |
| Caprylyl glycol | 0,460 ÷ 0,525 |
| Phenoxyethanol | 0,382 ÷ 0,436 |
| Polyacrylamide | 0,331 ÷ 0,378 |
| Tribehenin | 0,331 ÷ 0,378 |
| Thermus thermophilus ferment | 0,276 ÷ 0,315 |
| Glyceryl behenate | 0,221 ÷ 0,252 |
| Hydrogenated vegetable oil | 0,207 ÷ 0,236 |
| Sodium stearoyl lactylate | 0,184 ÷ 0,210 |
| Parfum | 0,175 ÷ 0,200 |
| C13-14 isoparaffin | 0,166 ÷ 0,189 |
| Ceramide 3 | 0,119 ÷ 0,135 |
| Lecithin | 0,117 ÷ 0,133 |
| Chlorphenesin | 0,092 ÷ 0,105 |
| Sodium dehydroacetate | 0,092 ÷ 0,105 |
| Sodium hyaluronate | 0,092 ÷ 0,105 |
| Sodium phytate | 0,092 ÷ 0,105 |
| Tetrasodium EDTA | 0,083 ÷ 0,095 |
| Candelilla cera | 0,069 ÷ 0,079 |
| Mica | 0,064 ÷ 0,074 |
| Alcohol denat. | 0,046 ÷ 0,053 |
| Citric acid | 0,046 ÷ 0,053 |
| Laureth-7 | 0,041 ÷ 0,047 |
| Cholesterol | 0,027 ÷ 0,030 |
| Stearic acid | 0,027 ÷ 0,030 |
| CI 77491 | 0,021 ÷ 0,024 |
| Lysophosphatidic acid | 0,018 ÷ 0,021 |
| Mannitol | 0,012 ÷ 0,014 |
| Sodium hydroxide | 0,009 ÷ 0,011 |
| CI 77891 | 0,005 ÷ 0,005 |
| Cyclotetrapeptide-24 Aminocyclohexane Carboxylate | 0,005 ÷ 0,005 |
| Lysolecithin | 0,003 ÷ 0,003 |
| Potassium sorbate | 0,003 ÷ 0,003 |
| Triethoxycaprylylsilane | 0,002 ÷ 0,002 |

**Emulsion 1**

| | |
|---|---|
| Aqua | 54,358 ÷ 62,039 |
| Glycerin | 6,129 ÷ 6,995 |
| Caprylic/Capric triglyceride | 3,680 ÷ 4,200 |
| Dimethicone | 3,680 ÷ 4,200 |
| Aluminum starch octenylsuccinate | 2,760 ÷ 3,150 |
| Butylene glycol | 2,760 ÷ 3,150 |
| Tribehenin PEG-20 ester | 2,760 ÷ 3,150 |
| Macadamia ternifolia seed oil | 1,840 ÷ 2,100 |
| Dipentaerythrityl hexacaprylate/Hexacaprate | 1,380 ÷ 1,575 |
| Cetyl alcohol | 1,104 ÷ 1,260 |
| Olus oil | 1,104 ÷ 1,260 |
| Cyclopentasiloxane | 0,920 ÷ 1,050 |
| Dimethiconol | 0,920 ÷ 1,050 |
| Hydrogenated lecithin | 0,920 ÷ 1,050 |
| PEG-20 methyl glucose sesquistearate | 0,920 ÷ 1,050 |
| Trehalose | 0,920 ÷ 1,050 |
| Ammonium acryloyldimethyltaurate/Beheneth-25 Methacrylate Crosspolymer | 0,552 ÷ 0,630 |
| Glyceryl dibehenate | 0,552 ÷ 0,630 |
| Ectoin | 0,465 ÷ 0,530 |
| Bis-Ethylhexyl Hydroxydimethoxy benzylmalonate | 0,460 ÷ 0,525 |
| Caprylyl glycol | 0,460 ÷ 0,525 |
| Phenoxyethanol | 0,382 ÷ 0,436 |
| Polyacrylamide | 0,331 ÷ 0,378 |
| Tribehenin | 0,331 ÷ 0,378 |
| Thermus thermophilus ferment | 0,276 ÷ 0,315 |
| Glyceryl behenate | 0,221 ÷ 0,252 |
| Hydrogenated vegetable oil | 0,207 ÷ 0,236 |
| Sodium stearoyl lactylate | 0,184 ÷ 0,210 |
| Parfum | 0,175 ÷ 0,200 |
| C13-14 isoparaffin | 0,166 ÷ 0,189 |
| Ceramide 3 | 0,119 ÷ 0,135 |
| Lecithin | 0,117 ÷ 0,133 |
| Chlorphenesin | 0,092 ÷ 0,105 |
| Sodium dehydroacetate | 0,092 ÷ 0,105 |
| Sodium hyaluronate | 0,092 ÷ 0,105 |
| Sodium phytate | 0,092 ÷ 0,105 |
| Tetrasodium EDTA | 0,083 ÷ 0,095 |
| Candelilla cera | 0,069 ÷ 0,079 |
| Mica | 0,064 ÷ 0,074 |
| Alcohol denat. | 0,046 ÷ 0,053 |
| Citric acid | 0,046 ÷ 0,053 |
| Laureth-7 | 0,041 ÷ 0,047 |
| Cholesterol | 0,027 ÷ 0,030 |
| Stearic acid | 0,027 ÷ 0,030 |
| CI 77491 | 0,021 ÷ 0,024 |
| Lysophosphatidic acid | 0,018 ÷ 0,021 |

**Emulsion 2**

| | |
|---|---|
| Aqua | 64,119 ÷ 73,180 |
| Glycerin | 7,044 ÷ 8,040 |
| Caprylic/Capric triglyceride | 2,760 ÷ 3,150 |
| Dimethicone | 2,760 ÷ 3,150 |
| Macadamia ternifolia seed oil | 1,840 ÷ 2,100 |
| Glyceryl stearate | 0,920 ÷ 1,050 |
| Hydrogenated lecithin | 0,920 ÷ 1,050 |
| PEG-100 stearate | 0,920 ÷ 1,050 |
| Propanediol dicaprylate | 0,920 ÷ 1,050 |
| Tapioca starch | 0,920 ÷ 1,050 |
| Trehalose | 0,920 ÷ 1,050 |
| Olus oil | 0,736 ÷ 0,840 |
| Cyclopentasiloxane | 0,690 ÷ 0,788 |
| Dimethiconol | 0,690 ÷ 0,788 |
| Ammonium acryloyldimethyltaurate/Beheneth-25 Methacrylate Crosspolymer | 0,644 ÷ 0,735 |
| Ectoin | 0,465 ÷ 0,530 |
| Butyrospermum parkii butter | 0,460 ÷ 0,525 |
| Caprylyl glycol | 0,460 ÷ 0,525 |
| hydroxy dimethoxybenzyl malonate | 0,460 ÷ 0,525 |
| Sodium stearoyl lactylate | 0,460 ÷ 0,525 |
| Phenoxyethanol | 0,382 ÷ 0,436 |
| Polyacrylamide | 0,294 ÷ 0,336 |
| Thermus thermophilus ferment | 0,276 ÷ 0,315 |
| Glyceryl dibehenate | 0,230 ÷ 0,263 |
| Parfum | 0,175 ÷ 0,200 |
| C13-14 isoparaffin | 0,147 ÷ 0,168 |
| Hydrogenated vegetable oil | 0,138 ÷ 0,158 |
| Tribehenin | 0,138 ÷ 0,158 |
| Lecithin | 0,117 ÷ 0,133 |
| Ceramide 3 | 0,105 ÷ 0,120 |
| Chlorphenesin | 0,092 ÷ 0,105 |
| Disodium EDTA | 0,092 ÷ 0,105 |
| Glyceryl behenate | 0,092 ÷ 0,105 |
| Sodium dehydroacetate | 0,092 ÷ 0,105 |
| Sodium hyaluronate | 0,092 ÷ 0,105 |
| Sodium phytate | 0,092 ÷ 0,105 |
| Citric acid | 0,074 ÷ 0,084 |
| Mica | 0,052 ÷ 0,059 |
| Alcohol denat. | 0,046 ÷ 0,053 |
| Candelilla cera | 0,046 ÷ 0,053 |
| Laureth-7 | 0,037 ÷ 0,042 |
| Lysophosphatidic acid | 0,018 ÷ 0,021 |
| CI 77491 | 0,017 ÷ 0,019 |
| Cholesterol | 0,013 ÷ 0,015 |
| Stearic acid | 0,013 ÷ 0,015 |
| Mannitol | 0,006 ÷ 0,007 |
| cyclopeptide 5 | 0,005 ÷ 0,005 |
| CI 77891 | 0,004 ÷ 0,004 |
| Lysolecithin | 0,003 ÷ 0,003 |
| Potassium sorbate | 0,003 ÷ 0,003 |

### Solar emulsion

| | |
|---|---|
| Aqua | 56,408 ÷ 64,379 |
| Butylene glycol dicaprylate/Dicaprate | 4,600 ÷ 5,250 |
| Ethylhexyl salicylate | 4,140 ÷ 4,725 |
| Butyl methoxydibenzoylmethane | 3,680 ÷ 4,200 |
| Ethylhexyl triazone | 3,680 ÷ 4,200 |
| C12-15 alkyl benzoate | 2,760 ÷ 3,150 |
| Dibutyl adipate | 2,760 ÷ 3,150 |
| Glycerin | 2,746 ÷ 3,134 |
| Bis-ethylhexyloxyphenol methoxyphenyl triazine | 2,300 ÷ 2,625 |
| Aluminum starch octenylsuccinate | 1,840 ÷ 2,100 |
| Ammonium acryloyldimethyltaurate/VP copolymer | 0,920 ÷ 1,050 |
| Octocrylene | 0,920 ÷ 1,050 |
| Potassium cetyl phosphate | 0,920 ÷ 1,050 |
| Triacontanyl PVP | 0,920 ÷ 1,050 |
| Trehalose | 0,920 ÷ 1,050 |
| Phenoxyethanol | 0,461 ÷ 0,526 |
| Allantoin | 0,460 ÷ 0,525 |
| Caprylyl glycol | 0,460 ÷ 0,525 |
| Olus oil | 0,460 ÷ 0,525 |
| PEG-30 dipolyhydroxystearate | 0,460 ÷ 0,525 |
| Ectoin | 0,465 ÷ 0,530 |
| Tocopheryl acetate | 0,460 ÷ 0,525 |
| Sodium dehydroacetate | 0,184 ÷ 0,210 |
| Bisabolol | 0,092 ÷ 0,105 |
| Disodium EDTA | 0,092 ÷ 0,105 |
| Propylene glycol | 0,056 ÷ 0,064 |
| Citric acid | 0,046 ÷ 0,053 |
| Sodium carboxymethyl betaglucan | 0,046 ÷ 0,053 |
| Aloe barbadensis leaf juice | 0,041 ÷ 0,047 |
| Sodium hyaluronate | 0,018 ÷ 0,021 |
| Parfum | 0,018 ÷ 0,021 |
| BHA | 0,009 ÷ 0,011 |
| Calendula officinalis extract | 0,009 ÷ 0,011 |
| Sorbitol | 0,009 ÷ 0,011 |
| Tocopherol | 0,009 ÷ 0,010 |
| Triethyl citrate | 0,009 ÷ 0,010 |
| Maltodextrin | 0,005 ÷ 0,005 |

### BIBLIOGRAPHY:

Lang KS, Lang PA, Bauer C et al. (2005). "Mechanisms of suicidal erythrocyte death". Cell. Physiol. Biochem. 15 (5): 195-202.
Kültz D, Burg M (1 November 1998). "Evolution of osmotic stress signaling via MAP kinase cascades". J. Exp. Biol. 201 (Pt 22): 3015-21.
Kültz D (2007). "Osmotic stress sensing and signaling in animals". FEBS journal 274 (22): 5781-5781.
"Intracellular water homeostasis and the mammalian cellular osmotic stress response". J. Cell. Physiol. 206 (1): 9-15.
Hélène Ollivier, Karine Pichavant-Rafini, Eneour Puill-Stephan, Patrick Calves, Liliane Nonnotte and Guy Nonnotte (2006). "Effects of hypo-osmotic stress on ATP release in isolated turbot (Scophthalmus maximus) hepatocytes". Biol. Cell 98 (7): 427-437.
Olivero P, Stutzin A. (2004). "Calcium modulates osmosensitive taurine efflux in HeLa cells". Neurochem Res. 29 (1): 169-76.
Lang F (October 2007). "Mechanisms and significance of cell volume regulation". J Am Coll Nutr 26 (5 Suppl): 613S-623S. Sussich F, Skopec C, Brady J, Cesàro A (August 2001). "Reversible dehydration of trehalose and anhydrobiosis: from solution state to an exotic crystal?". Carbohydr. Res. 334 (3): 165-76.
Crowe JH, Carpenter JF, Crowe LM (1998). "The role of vitrification in anhydrobiosis". Annu. Rev. Physiol. 60: 73-103.
Hara-Chikuma M, Verkman AS. Roles of aquaporin-3 in the epidermis. J Invest Dermatol. 2008 Sep;128(9):2145-51.
Boury-Jamot M, Daraspe J, Bonté F, Perrier E, Schnebert S, Dumas M, Verbavatz JM. Skin aquaporins: function in hydration, wound healing, and skin epidermis homeostasis. Handb Exp Pharmacol. 2009 ;(190):205-17.
Sougrat R, Morand M, Gondran C, Barré P, Gobin R, Bonté F, Dumas M, Verbavatz JM. Functional expression of AQP3 in human skin epidermis and reconstructed epidermis.
J Invest Dermatol. 2002 Apr;118(4):678-85.
Hee-Sae Park et al. Heat Shock Protein Hsp72 Is a Negative Regulator of Apoptosis Signal-Regulating Kinase 1. Mol Cell Biol. 2002 November; 22(22): 7721-7730. Alexander Asea. Mechanism of HSP72 release. J. Biosci. 2007, 32 579-584.
GE Pierard : EEMCO GUIDANCE FOR THE MEASUREMENT OF SKIN MICROCIRCULATION" Skin Pharmacol. Appl. Skin. Physiol.; 15(6) :442-456 (Nov. -Dec. 2002).
J. Sondergaard, J. Serup and G. Tikjob "ULTRASONIC A AND B-SCANNING IN CLINICAL AND EXPERIMENTAL DERAMTOLOGY" Acta
Dermatovener (Stockh)Suppl.120 76-82. J. Serup "TEN YEARS' EXPERIENCE WITH HIGH FREQUENCY ULTRASOUND EXAMINATION OF THE SKIN: DEVELOPMENT AND
REFINEMENT OF THE TECHNIQUE AND EQUIPMENT" Ultrasound in dermatology (1992)41-54.
Guidelines for the evaluation of the efficacy of cosmetic products COLIPA Guide lines, May 2008.
S. Seidenari "DIAGNOSTICA NON INVASIVA IN DERMATOLOGIA" ed. EDRA (1998)
J. Serup, G.B.E. Jemec: "HANDBOOK OF NON -INVASIVE METHODS AND THE SKIN" CRC Press. Inc., (1995)

## Claims

1. Cosmetic compositions comprising as the active ingredients an association of ectoine and trehalose in total amounts ranging from 0,5 to 2% based on the total composition weight in combination with suitable excipients and/or diluents.

2. The cosmetic composition according to claim 1 for dermal applications.

3. The cosmetic compositions according to claim 1 or 2 containing ectoine and trehalose in molar ratios ranging from 1:5 to 5:1

4. The cosmetic compositions according to claim 3 wherein said molar ratio range from 1:4 to 4:1.

5. The cosmetic compositions according to claim 4 wherein said molar ratio range from 1:3 to 3:1.

6. The cosmetic compositions according to claim 5 wherein said molar ratio range from 1:2 to 2:1.

7. The cosmetic compositions according to claim 6, wherein said total amounts range from 1 to 1,5% based on the total composition weight.

8. The cosmetic compositions according to anyone of claims 1-7 further comprising at least one active ingredient selected from Ceramide, cholesterol, fatty acids, vitamins, peptides, hyaluronic acid, Lysolecithins, lysophoshatidic acid, ferment lysates and mixtures thereof.

9. The cosmetic compositions according to any one of claims 1-8 in the form w/o or o/w emulsions, sera ointment, gel-creams, body milks, sun and after sun milks, sun and after sun gel-cream and sun sera.

## Patentansprüche

1. Kosmetische Zusammensetzungen, umfassend als aktive Inhaltsstoffe eine Assoziation aus Ectoin und Trehalose in Gesamtgehalten, die basierend auf dem Gesamtgewicht der Zusammensetzung von 0,5 bis 2 % reichen, in Kombination mit geeigneten Hilfsstoffen und/oder Verdünnungsmitteln.

2. Kosmetische Zusammensetzung nach Anspruch 1 für dermale Anwendungen.

3. Kosmetische Zusammensetzungen nach Anspruch 1 oder 2, enthaltend Ectoin und Trehalose in molaren Verhältnissen, die von 1:5 bis 5:1 reichen.

4. Kosmetische Zusammensetzungen nach Anspruch 3, wobei das molare Verhältnis von 1:4 bis 4:1 reicht.

5. Kosmetische Zusammensetzungen nach Anspruch 4, wobei das molare Verhältnis von 1:3 bis 3:1 reicht.

6. Kosmetische Zusammensetzungen nach Anspruch 5, wobei das molare Verhältnis von 1:2 bis 2:1 reicht.

7. Kosmetische Zusammensetzungen nach Anspruch 6, wobei die Gesamtgehalte basierend auf dem Gesamtgewicht der Zusammensetzung von 1 bis 1,5 % reichen.

8. Kosmetische Zusammensetzungen nach einem der Ansprüche 1 bis 7, ferner umfassend wenigstens einen aktiven Inhaltsstoff, der aus Ceramid, Cholesterin, Fettsäuren, Vitaminen, Peptiden, Hyaluronsäure, Lysolecithinen, Lysophosphatidsäure, Fermentlysaten und Mischungen davon ausgewählt ist.

9. Kosmetische Zusammensetzungen nach einem der Ansprüche 1 bis 8 in der Form von W/O- oder O/W-Emulsionen, Serensalbe, Gelcremen, Körperlotionen, Sonnen- und After-Sun-Lotionen, Sonnen- und After-Sun-Gelcremen und Sonnenseren.

## Revendications

1. Compositions cosmétiques comprenant, en tant qu'ingrédients actifs, une association d'ectoïne et de tréhalose, en teneurs totales comprises entre 0,5 et 2 % sur la base du poids total de la composition, en combinaison avec des excipients et/ou diluants appropriés.

2. Composition cosmétique selon la revendication 1, pour des applications dermiques.

3. Compositions cosmétiques selon la revendication 1 ou 2, contenant de l'ectoïne et du tréhalose dans des rapports molaires allant de 1: 5 à 5:1.

4. Compositions cosmétiques selon la revendication 3, dans lesquelles le rapport molaire va de 1:4 à 4:1.

5. Compositions cosmétiques selon la revendication 4, dans lesquelles le rapport molaire va de 1:3 à 3:1.

6. Compositions cosmétiques selon la revendication 5, dans lesquelles le rapport molaire va de 1:2 à 2:1.

7. Compositions cosmétiques selon la revendication 6, dans lesquelles les teneurs totales sont comprises entre 1 et 1,5 % sur la base du poids total de la composition.

8. Compositions cosmétiques selon l'une quelconque des revendications 1 à 7, comprenant en outre au moins un ingrédient actif choisi parmi la céramide, le cholestérol, les acides gras, les vitamines, les peptides, l'acide hyaluronique, les lysolécithines, l'acide lysophosphatidique, les lysats de ferment et les mélanges de ceux-ci.

9. Compositions cosmétiques selon l'une quelconque des revendications 1 à 8, sous la forme d'émulsions E/H ou H/E, d'onguent de sérums, de crèmes gels, de laits corporels, de laits solaires et après-soleil, de crèmes gels solaires et après-soleil et de sérums solaires.
